# EUROPÄISCHE PATENTSCHRIFT

(11) **EP 3 253 271 B1**
(45) Veröffentlichungstag und Bekanntmachung des Hinweises auf die Patenterteilung: **06.03.2019**
(21) Anmeldenummer: 16700813.5
(22) Anmeldetag: 15.01.2016
(51) Int. Cl.: A61B 1/00, G02B 23/24, G02B 25/00, G02B 27/00

(54) **OKULAR FÜR EIN CHIRURGISCHES INSTRUMENT**
EYEPIECE FOR A SURGICAL INSTRUMENT
OCULAIRE POUR UN INSTRUMENT CHIRURGICAL

(30) Priorität: 06.02.2015 DE 102015202137
(43) Veröffentlichungstag der Anmeldung: 13.12.2017
(73) Patentinhaber: Olympus Winter&Ibe GmbH, 22045 Hamburg (DE)
(72) Erfinder: KIEDROWSKI, Gregor, 22397 Hamburg (DE)
(74) Vertreter: Seemann & Partner Patentanwälte mbB
(86) Internationale Anmeldenummer: PCT/EP2016/050752
(87) Internationale Veröffentlichungsnummer: WO 2016/124370

(56) Entgegenhaltungen:
- JP-A- S5 895 317
- JP-A- S6 015 618
- US-A- 6 025 873

## Beschreibung

Die Erfindung betrifft eine Okulareinrichtung für ein chirurgisches Instrument, insbesondere Endoskop oder Laparoskop, mit einer Okularfassung für eine optische Baugruppe, wobei ein Planglas in einer Halterung aufgenommen ist und wobei die Halterung für das Planglas mit der Okularfassung verbindbar oder verbunden ist.

Außerdem betrifft die Erfindung ein chirurgisches Instrument, insbesondere Endoskop oder Laparoskop.

Minimal-invasive endoskopische Eingriffe am menschlichen oder tierischen Körper erfolgen mittels Endoskopen mit langgestrecktem bzw. längserstrecktem Schaft, die durch eine vorhandene oder vor dem Eingriff zu diesem Zweck erzeugte Körperöffnung in einen Körperinnen- bzw. -hohlraum eingeführt werden. Da eine direkte Sicht von außen auf das Operationsfeld im Körperhohlraum nicht möglich ist, erlauben bekannte Endoskope einen Blick in den zu behandelnden Körperhohlraum. Hierzu weisen übliche Endoskope eine Optik mit einer oder mehreren Linsen an der distalen Spitze des Endoskopschafts auf, die Licht aus dem Körperhohlraum in das Endoskop hinein lenken. Der Endoskopschaft kann eine Anordnung von Linsen, beispielsweise Stablinsen, aufweisen, mittels denen Licht aus dem Körperhohlraum hinaus zum proximalen Ende des Endoskops geleitet wird, d.h. zu dem Ende, das von einem Operateur oder Chirurgen gehalten und bedient wird.
Im proximalen Bereich des Endoskops, beispielsweise an einem Griff, befindet sich ein Okulartrichter mit einem Okular, also einer Optik, aus der das Licht, das an der distalen Spitze des Endoskops eintritt, wieder austritt. Ein solches Okular kann zur direkten Beobachtung mit bloßem Auge verwendet werden, das an den Okulartrichter herangeführt wird. Das Dokument JP S60 15618 A beispielsweise offenbart eine Okulareinrichtung mit einem Planglas gemäß dem Oberbegriff des unabhängigen Anspruchs 1. Ausgehend von diesem Stand der Technik besteht die Aufgabe der Erfindung darin, bei chirurgischen Instrumenten eine reflektionsfreie Erfassung von Bildern, insbesondere mittels einer Bildsensoreinheit, zu ermöglichen, wobei der konstruktive Aufwand möglichst gering gehalten werden soll.
Gelöst wird diese Aufgabe durch eine Okulareinrichtung für ein chirurgisches Instrument, insbesondere Endoskop oder Laparoskop, mit einer Okularfassung für eine optische Baugruppe, wobei ein Planglas in einer Halterung aufgenommen ist und wobei die Okularfassung mit der Halterung für das Planglas verbindbar oder verbunden ist, die dadurch weitergebildet wird, dass am Planglas ein verbreiteter Seitenrand mit einer Anlagefläche für die Okularfassung und einer Anlagefläche für die Halterung vorgesehen ist, wobei die Oberflächennormale des Planglases und die der Okularfassung zugewandte Anlagefläche des Planglases, vorzugsweise bei Aufnahme des Planglases in der Halterung, in einem Winkel ungleich 0°, vorzugsweise in einem Winkel zwischen 2,0° bis 10,0°, zueinander ausgerichtet sind.

Die Erfindung beruht auf dem Gedanken, dass bei der Okulareinrichtung eines chirurgischen Instruments die Oberflächennormale des Planglases, die der Okularfassung zugewandt ist, in Bezug auf die optische Achse der in der Okularfassung aufgenommenen optischen Baugruppe in einem Winkel ungleich 0° (d.h. ≠ 0°), d.h. nicht parallel zueinander, ausgerichtet wird. Hierdurch werden die Eintrittsseite und die Austrittsseite bzw. die Oberflächen des Planglases in Bezug auf die senkrechte Ebene zur optischen Achse der Okulareinrichtung in einem Winkel ungleich 0° ausgerichtet, wodurch bei Durchtritt von Lichtstrahlen durch das Planglas bzw. das Okularglas keine Reflektionen innerhalb des Planglases entstehen und mögliche Artefakte aufgrund von (Licht-)Reflektionen in einem Bilderfassungssensor vermieden werden.

Ferner ist es dabei vorgesehen, dass bei Anordnung des Planglases in der Okularfassung durch den verbreiterten Seitenrand des Planglases eine Anlagefläche am Planglas vorgesehen ist, die senkrecht zur optischen bzw. mechanischen Achse der Okularfassung bzw. zur optischen Achse der optischen Baugruppe der Okularfassung ausgerichtet ist.

Das Planglas ist als flaches Okularfenster für das Okular ausgebildet und weist zwei parallele Oberflächen auf, durch die die Lichtstrahlen hindurchtreten. Die Oberflächen des Planglases weisen jeweils eine entsprechende Oberflächennormale auf, die senkrecht zur planen Oberfläche des Planglases sind. Vorzugsweise ist das Planglas aus Saphir herstellt.

Das als Okularfenster vorgesehene Planglas ist vorzugsweise mit einem umlaufenden verbreiterten Seitenrand ausgebildet, wobei die durch den Seitenrand bereitgestellte Anlagefläche für die Okularfassung in einem Winkel zu den Oberflächen des Planglases ausgerichtet ist, der dem Winkel zwischen der Oberflächennormale des Planglases und der optischen Achse der in der Okularfassung aufgenommenen optischen Baugruppe entspricht.

Bei Anordnung des Planglases an der mit einer optischen Baugruppe versehenen Okularfassung ist eine plane Anschlagschulter für das Planglas ausgebildet, wobei die parallel zueinander ausgerichteten Oberflächen des Planglases schräg zur ansonsten senkrechten Ausrichtung des Planglases (gemäß dem Stand der Technik) angeordnet werden. Der verbreiterte Seitenrand des Planglases ist hierbei als plane Anschlagschulter ausgebildet, wobei die plane Anschlagschulter als verbreiterter Seitenrand in einem spitzen Winkel zu einer oder beiden Oberflächen des Planglases ausgebildet ist.

Dazu ist in einer Weiterbildung vorgesehen, dass die Oberflächennormale oder Oberflächennormalen des Planglases und die der Okularfassung zugewandte Anlagefläche des Planglases in einem Winkel zwischen 4,0° bis 8,0°, insbesondere in einem Winkel von 6°, zueinander ausgerichtet sind. Entsprechend ist der verbreiterte Seitenrand des Planglases mit der Anlagefläche für die Okularfassung in einem Winkel von 4,0° bis 8,0°, insbesondere in einem Winkel von 6°, zum Beispiel zur Oberfläche an der distalen Eintrittsseite des Planglases ausgebildet.

Ferner ist es bei der Okulareinrichtung bevorzugt, dass der verbreiterte, vorzugsweise umlaufende, Seitenrand des Planglases bzw. des Okularglases in eine Aufnahme der Halterung form- und funktionskomplementär aufgenommen ist. Das Planglas kann in einer Ausgestaltung zylinderförmig ausgebildet sein.

Insbesondere ist der verbreiterte Seitenrand des Planglases ringartig ausgebildet.

Um eine zuverlässige Verbindung zwischen der Okularfassung, in der eine optische Baugruppe aufgenommen ist, und der Halterung zu erreichen, ist vorzugsweise die Halterung als Überwurfmutter, vorzugsweise mit einem Innengewinde, ausgebildet. Die als Überwurfmutter mit einem Innengewinde bereitgestellte Halterung wirkt mit einem Außengewinde an der Okularfassung zusammen. Die in der Okularfassung aufgenommene optische Baugruppe weist eine oder mehrere Linsen und gegebenenfalls weitere optische Elemente auf.

Des Weiteren ist es bevorzugt, dass die Halterung aus Kunststoff hergestellt ist, wobei die Halterung in der Ausbildung als Überwurfmutter eine zum Planglas passende Innenschräge aufweist, die einen Überstand des Planglases bzw. Okularglases abdeckt sowie ausgleicht.

Außerdem zeichnet sich eine Ausführungsform der Okulareinrichtung dadurch aus, dass die Halterung, insbesondere Überwurfmutter, auf der vom Planglas abgewandten Seite mit einer Mulde ausgebildet ist. Durch die proximalseitig ausgebildete Mulde wird erreicht, dass ein bündiger Abschluss mit dem schräg angestellten Planglas bzw. Okularglas ausgebildet ist, wodurch auf der proximalen Lichtaustrittsseite Schmutzkanten oder dergleichen zwischen Planglas und Halterung vermieden werden.

Insbesondere ist bei der Okulareinrichtung des chirurgischen Instruments vorgesehen, dass die der Okularfassung abgewandte, d.h. proximale Seite des Planglases bündig mit dem das Planglas umgebenden Bereich oder Randbereich der Halterung, insbesondere mit dem das Planglas umgebenden Randbereich der Mulde der Halterung, ausgerichtet ist.

Außerdem ist bei der Okulareinrichtung vorgesehen, dass ein Okulartrichter an der Halterung angeordnet oder anordbar ist. Hierbei wird der Okulartrichter beispielsweise mit dem Gehäuse für die Okularfassung verschraubt, wobei der Okulartrichter innenseitig an die Halterung bzw. die Überwurfmutter angepasst ist und weiterhin vorzugsweise mit einem Einstich für eine radiale Dichtung (z.B. für einen O-Ring) ausgebildet ist.

Darüber hinaus wird die Aufgabe gelöst durch ein chirurgisches Instrument, insbesondere Endoskop oder Laparoskop, das mit einer voranstehend beschriebenen Okulareinrichtung ausgebildet ist. Zur Vermeidung von Wiederholungen wird auf die obigen Ausführungen verwiesen. Unter Verwendung einer erfindungsgemäßen Okulareinrichtung werden Reflektionen im Planglas bei der Aufnahme mit einer Bildsensoreinrichtung sowie Geisterbilder aufgrund von Reflektionen durch das Okularfenster bzw. das proximale Planglas des Okulars vermieden.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Seitenansicht eines Endoskops,
- Fig. 2: schematisch einen Querschnitt durch ein Okular eines Endoskops im Ausschnitt und
- Fig. 3: schematisch eine Seitenansicht eines als Okularfenster ausgebildetes Planglas.

In den Zeichnungen sind jeweils gleiche oder gleichartige Elemente und/oder Teile mit denselben Bezugsziffern versehen, so dass von einer erneuten Vorstellung jeweils abgesehen wird.

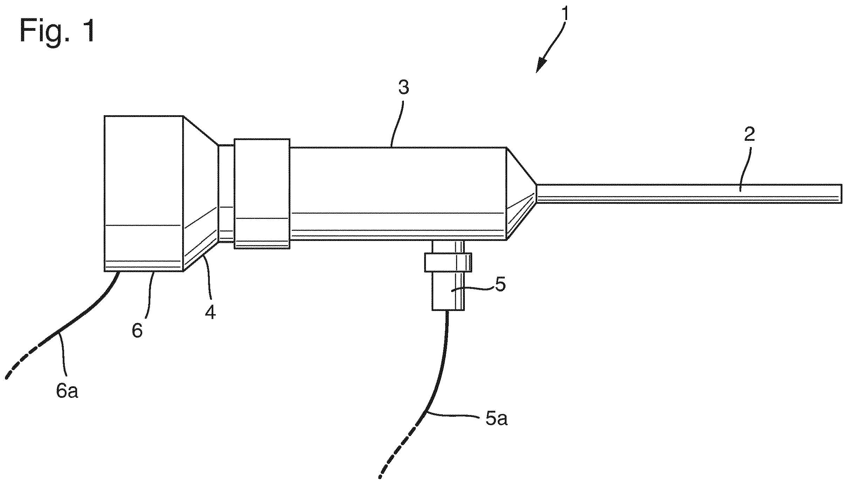

Fig. 1 zeigt eine schematische Seitenansicht eines Endoskops 1. das am distalen Ende einen rohrförmigen Schaft 2 mit einer Optik aufweist. Dieser rohrförmige Schaft 2 wird bei einem minimalinvasiven Eingriff oder einer minimalinvasiven Untersuchung durch eine Körperöffnung in einen Körperhohlraum eingeführt. Der rohrförmige Schaft 2 mündet in ein Gehäuse 3, das wiederum am proximalen Ende, d.h. am Ende, das zum Chirurgen oder Operateur hin angeordnet ist, in einen Okulartrichter 4 mit einem nicht dargestellten Okular mündet. Das Gehäuse 3 dient auch der Handhabung des Endoskops 1.

Seitlich ist am Gehäuse 3 des Endoskops 1 eine Lichtquelle 5, insbesondere eine LED-Lichtquelle, angeordnet, die von der Seite helles Licht in die Optik des Endoskops 1 einleitet, von wo das eingeleitete Licht am distalen Ende, d.h. an der Spitze des rohrförmigen Schafts 2 austritt, um ein Operationsfeld auszuleuchten. Die Lichtquelle 5 weist ein Anschlusskabel 5a auf. Im Falle einer herkömmlichen Optik kann es sich bei der Lichtquelle 5 um einen Adapter handeln, an den ein Glasfaserbündel als Anschlusskabel 5a angefügt wird. Das durch das Glasfaserbündel gelieferte Licht wird dann mittels des Adapters in das Endoskop 1 eingeleitet. In einer alternativen Version kann es sich um eine aktive Lichtquelle 5, beispielsweise auf der Grundlage von LEDs, Halogenleuchtkörpern oder dergleichen handeln, wobei in diesem Fall das Anschlusskabel 5a ein Stromversorgungskabel ist.

Am Okulartrichter 4 des Endoskops 1 ist ein schematisch dargestellter Kamerakopf 6 mit einem nicht dargestellten Okularadapter angeordnet, der das aus dem Okular des Endoskops 1 austretende Licht mit einer eigenen Optik erfasst und auf einen optischen Flächensensor, beispielsweise einen CCD-Chip, fokussiert. Mittels des Anschlusses 6a für den Kamerakopf 6 wird der Kamerakopf 6 mit Strom versorgt, werden Bildsignale von dem Flächensensor an eine externe Auswerteeinheit übermittelt und Steuersignale an den Kamerakopf 6 übermittelt.

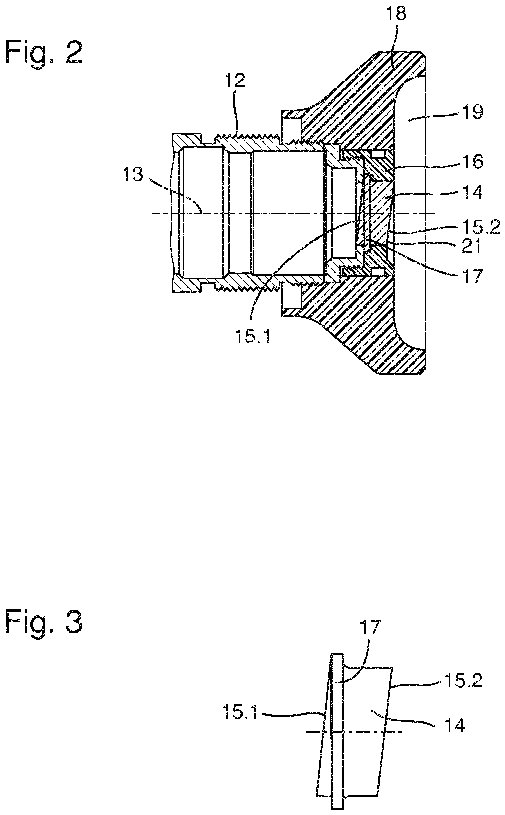

In Fig. 2 ist schematisch ein Querschnitt durch ein Okular am proximalseitigen Ende des Endoskops im Ausschnitt dargestellt. Hierbei weist das Okular eine Okularfassung 12 auf, in der z.B. (hier nicht dargestellte) abbildende Linsen als optische Baugruppe angeordnet sind. Das Okular weist eine optische Achse 13 der optischen Baugruppe auf.

Am proximalen Ende der Okularfassung 12, die auch als Okularfensterfassung bezeichnet wird, ist als Okularfenster ein Planglas 14 angeordnet, das in einer mit einer Ausnehmung versehenen Überwurfmutter 16 aufgenommen ist. Die Überwurfmutter 16 weist ein Innengewinde auf, das mit einem Außengewinde der Okularfassung 12 zusammenwirkt. Darüber hinaus ist an der proximalen Seite des Endoskops an der Okularfassung 12 ein Okulartrichter 18 angeordnet, der eine Trichtermulde 19 aufweist.

Das von der Überwurfmutter 16 gehalterte Planglas 14 weist eine Lichteintrittsseite 15.1 und eine Lichtaustrittsseite 15.2 auf, die parallel zueinander ausgerichtet sind. Die Lichteintrittsseite 15.1 und die Lichtaustrittsseite 15.2 sind in einem Winkel ≠ 90° zur optischen Achse 13 ausgerichtet. Die optische Achse 13 ist kollinear zur mechanischen Achse des Okulars.

Um die Oberflächennormale des Planglases 14 im Winkel von 6° zur optischen Achse auszurichten, weist das Planglas 14 eine Halteschulter 17 auf. Durch die umlaufende Halteschulter 17 als plane Anschlagschulter wird eine Planfläche zur Anlage an die proximale Öffnung der Okularfassung 12 bereitgestellt. Die Halteschulter 17 ist hierbei in einem Winkel von 6° zur Ebene der Lichteintrittsseite 15.1 bzw. zur Lichtaustrittsseite 15.2 des Planglases 14 ausgerichtet. Dadurch sind die Oberflächennormalen der parallelen Lichteintrittsseite 15.1 und Lichtaustrittsseite 15.2 des Planglases 14 im Winkel von 6° zur optischen Achse 13 ausgerichtet.

In Fig. 3 ist schematisch eine vergrößerte Ansicht des erfindungsgemäßen Planglases 14 dargestellt.

Die von der Halteschulter 17 zur Okularfassung 12 weisende Anlageschulter ist hierbei eine Planfläche, die senkrecht zur optischen Achse 13 bzw. zur mechanischen Achse der Okularfassung ausgerichtet ist. Die proximale Anlagefläche der Halteschulter 17 ist hierbei in einer formkomplementären Ausnehmung der Überwurfmutter 16 angeordnet.

Die Überwurfmutter 16 ist vorzugsweise aus Kunststoff hergestellt und hat weiterhin vorzugsweise eine zur Lichtaustrittsseite 15.2 des Planglases 14 ausgebildete Innenschräge, wodurch die Überwurfmutter 16 proximalseitig eine Mulde 21 aufweist. Dabei ist die Überwurfmutter 16 proximalseitig so ausgebildet, dass die Mulde 21 mit dem an das Planglas 14 angrenzenden Bereich und die proximale Oberfläche, d.h. die Lichtaustrittsseite 15.2 des Planglases 14 bündig ausgerichtet sind. Durch die bündige Ausrichtung der proximalen Lichtaustrittsseite 15.2 des Planglases 14 mit dem an das Planglas 14 angrenzenden und umgebenden Randbereich der Mulde 21 wird ein störungskantenfreier Übergang zwischen dem Rand des Planglases 14 und der Mulde 21 ausgebildet, wodurch ein Höhensprung zwischen dem Planglas 14 und der das Planglas 14 umgebenden Mulde 21 vermieden wird.

### Bezugszeichenliste

- 1: Endoskop
- 2: rohrförmiger Schaft mit Optik
- 3: Gehäuse
- 4: Okulartrichter
- 5: Lichtquelle
- 5a: Anschlusskabel für Lichtquelle
- 6: Kamerakopf
- 6a: Anschluss für den Kamerakopf
- 12: Okularfassung
- 14: Planglas
- 15.1: Lichteintrittsseite
- 15.2: Lichtaustrittsseite
- 16: Überwurfmutter
- 17: Halteschulter
- 18: Okulartrichter
- 19: Trichtermulde
- 21: Mulde

## Patentansprüche

1. Okulareinrichtung für ein chirurgisches Instrument (1), insbesondere Endoskop (1) oder Laparoskop, mit einer Okularfassung (12) für eine optische Baugruppe, wobei ein Planglas (14) in einer Halterung aufgenommen ist und wobei die Halterung (16) für das Planglas (14) mit der Okularfassung (12) verbindbar oder verbunden ist, **dadurch gekennzeichnet, dass** am Planglas (14) ein verbreiteter Seitenrand (17) mit einer Anlagefläche für die Okularfassung (12) und mit einer Anlagefläche für die Halterung vorgesehen ist, wobei die Oberflächennormale des Planglases (14) und die der Okularfassung (12) zugewandte Anlagefläche des Planglases (14) in einem Winkel ungleich 0°, vorzugsweise in einem Winkel zwischen 2,0° bis 10,0°, zueinander ausgerichtet sind.

2. Okulareinrichtung nach Anspruch 1, **dadurch gekennzeichnet, dass** die Oberflächennormale des Planglases (14) und die der Okularfassung (12) zugewandte Anlagefläche des Planglases (14) in einem Winkel zwischen 4,0° bis 8,0°, insbesondere in einem Winkel von 6°, zueinander ausgerichtet sind.

3. Okulareinrichtung nach Anspruch 1 oder 2, **dadurch gekennzeichnet, dass** der verbreiterte Seitenrand (17) des Planglases (14) in einer Aufnahme der Halterung (16) form- und funktionskomplementär aufgenommen ist.

4. Okulareinrichtung nach einem der Ansprüche 1 bis 3, **dadurch gekennzeichnet, dass** der verbreiterte, vorzugsweise umlaufende, Seitenrand (17) des Planglases (14) ringartig ausgebildet ist.

5. Okulareinrichtung nach einem der Ansprüche 1 bis 4, **dadurch gekennzeichnet, dass** die Halterung (16) als Überwurfmutter (16), vorzugsweise mit einem Innengewinde, ausgebildet ist.

6. Okulareinrichtung nach einem der Ansprüche 1 bis 5, **dadurch gekennzeichnet, dass** die Halterung (16) aus Kunststoff hergestellt ist.

7. Okulareinrichtung nach einem der Ansprüche 1 bis 6, **dadurch gekennzeichnet, dass** die Halterung (16), insbesondere Überwurfmutter, auf der vom Planglas (14) abgewandten Seite mit einer Mulde (21) ausgebildet ist.

8. Okulareinrichtung nach Anspruch 7, **dadurch gekennzeichnet, dass** die der Okularfassung (12) abgewandte Seite des Planglases (14) bündig mit dem das Planglas (14) umgebenden Bereich der Halterung (16), insbesondere mit dem das Planglas (14) umgebenden Randbereich der Mulde (21), ausgerichtet ist.

9. Okulareinrichtung nach einem der Ansprüche 1 bis 8, **dadurch gekennzeichnet, dass** ein Okulartrichter (18) an der Halterung (16) angeordnet oder anordbar ist.

10. Chirurgisches Instrument (1), insbesondere Endoskop (1) oder Laparoskop, mit einer Okulareinrichtung nach einem der Ansprüche 1 bis 9.

## Claims

1. An eyepiece device for a surgical instrument (1), in particular an endoscope (1) or laparoscope, with an ocular mount (12) for an optical assembly, wherein an optical flat (14) is accommodated in a holder, and wherein the holder (16) for the optical flat (14) is connected to the ocular mount (12), **characterized in that** a widened side edge (17) on the optical flat (14) is provided with a contact surface for the ocular mount (12) and with a contact surface for the holder, wherein the surface normal of the optical flat (14) and the contact surface of the optical flat (14) facing the ocular mount (12) are aligned at an angle different than 0°, preferably an angle between 2.0° and 10.0°, relative to each other.

2. The eyepiece device according to claim 1, **characterized in that** the surface normal of the optical flat (14) and the contact surface of the optical flat (14) facing the ocular mount (12) are aligned at an angle between 4.0° and 8.0°, in particular at an angle of 6°, relative to each other.

3. The eyepiece device according to claim 1 or 2, **characterized in that** the widened side edge (17) of the optical flat (14) is accommodated in a seat in the holder (16) in a manner complementary to the shape and function.

4. The eyepiece device according to one of claims 1 to 3, **characterized in that** the widened, preferably peripheral side edge (17) of the optical flat (14) is designed in a ring shape.

5. The eyepiece device according to one of claims 1 to 4, **characterized in that** the holder (16) is designed as a union nut (16), preferably with an internal thread.

6. The eyepiece device according to one of claims 1 to 5, **characterized in that** the holder (16) is made of plastic.

7. The eyepiece device according to one of claims 1 to 6, **characterized in that** the holder (16), in particular the union nut, is designed with a recess (21) in the side facing away from the optical flat (14).

8. The eyepiece device according to claim 7, **characterized in that** the side of the optical flat (14) facing away from the ocular mount (12) is aligned flush with the region of the holder (16) surrounding the optical flat (14), in particular with the edge region of the recess (21) surrounding the optical flat (14).

9. The eyepiece device according to one of claims 1 to 8, **characterized in that** an ocular funnel (18) is arranged or arrangeable on the holder (16).

10. A surgical instrument (1), in particular an endoscope (1) or laparoscope, with an eyepiece device according to one of claims 1 to 9.

## Revendications

1. Dispositif formant oculaire pour un instrument chirurgical (1), en particulier un endoscope (1) ou un laparoscope, ayant une monture oculaire (12) pour un ensemble optique, dans lequel un verre à faces planes (14) est reçu dans un support et dans lequel le support (16) pour le verre à faces planes (14) est apte à être relié ou est relié à la monture oculaire (12), **caractérisé en ce que** le verre à faces planes (14) a un bord latéral élargi (17) pourvu d'une surface de contact pour la monture oculaire (12) et d'une surface de contact pour le support, dans lequel la normale à la surface du verre à faces planes (14) et la surface de contact dirigée vers la monture oculaire (12) du verre à faces planes (14) forment l'une par rapport à l'autre un angle différent de 0°, de préférence compris entre 2,0° et 10,0°.

2. Dispositif formant oculaire selon la revendication 1, **caractérisé en ce que** la normale à la surface du verre à faces planes (14) et la surface de contact dirigée vers la monture oculaire (12) du verre à faces planes (14) forment l'une par rapport à l'autre un angle compris entre 4,0° et 8,0°, en particulier un angle de 6°.

3. Dispositif formant oculaire selon la revendication 1 ou la revendication 2, **caractérisé en ce que** le bord latéral élargi (17) du verre à faces planes (14) est reçu par complémentarité de formes et de fonction dans un logement du support (16).

4. Dispositif formant oculaire selon l'une des revendications 1 à 3, **caractérisé en ce que** le bord latéral élargi (17), de préférence circonférentiel, du verre à faces planes (14) est en forme d'anneau.

5. Dispositif formant oculaire selon l'une des revendications 1 à 4, **caractérisé en ce que** le support (16) a une forme d'écrou-union (16) ayant de préférence un filetage intérieur.

6. Dispositif formant oculaire selon l'une des revendications 1 à 5, **caractérisé en ce que** le support (16) est fabriqué à partir d'une matière plastique.

7. Dispositif formant oculaire selon l'une des revendications 1 à 6, **caractérisé en ce que** le support (16), en particulier l'écrou-union, est formé sur le côté opposé au verre à faces planes (14) avec un évidement (21).

8. Dispositif formant oculaire selon la revendication 7, **caractérisé en ce que** le côté du verre à faces planes (14) opposé à la monture oculaire (12) est aligné en affleurant la zone du support (16) entourant le verre à faces planes (14), en particulier avec la zone de bord de l'évidement (21) entourant le verre à faces planes (14).

9. Dispositif formant oculaire selon l'une des revendications 1 à 8, **caractérisé en ce qu'**un oculaire (18) est agencé ou est apte à être agencé sur le support (16).

10. Instrument chirurgical (1), en particulier un endoscope (1) ou un laparoscope, ayant un dispositif formant oculaire selon l'une des revendications 1 à 9.
